# EUROPEAN PATENT APPLICATION

(11) **EP 2 732 761 A1**
(43) Date of publication of application: **21.05.2014**
(21) Application number: 13192728.7
(22) Date of filing: 13.11.2013
(51) Int. Cl.: A61B 5/11, G06F 19/00, G02B 27/01

(54) **Augmented reality system in the patient care environment**

(30) Priority: 14.11.2012 US 201261726565 P
(71) Applicant: Hill-Rom Services, Inc., Batesville, IN 47006-9167 (US)
(72) Inventor: Ribble, David, Indianapolis, IN Indiana 46202 (US); McCleerey, Michelle, Raleigh, NC North Carolina 27604 (US); Agdeppa, Eric, Cincinnati, OH 45249 (US)
(74) Representative: Findlay, Alice Rosemary

(57) **Abstract**

An augmented reality system (10) comprises a wearable user interface system (12), a care facility network (38), and a medical device (16). The network is in communication with the user interface system. The medical device is configured to be used with a patient and is in communication with the user interface system. The user interface system receives information from the care facility network and the medical device and displays the information in a user's field of vision. Based on the position of the user interface system and the respective positions of persons and/or medical devices retrieved from a location database, information on a person and/or a medical device proximate to the user interface system is provided to the user interface system.

## Description

This disclosure relates to augmented reality systems in the patient care environment. More particularly, but not exclusively, one contemplated embodiment relates to an augmented reality devices for use with person support structures and other hospital equipment. While various systems may have been developed, there is still room for improvement. Thus, a need persists for further contributions in this area of technology.

An augmented reality system comprises a user interface system, a care facility network, and a medical device. The network is in communication with the user interface system. The medical device is configured to be used with a patient and is in communication with the user interface system. The user interface system receives information from the care facility network and the medical device and displays the information in a user's field of vision.

The invention will now be further described by way of example with reference to the accompanying drawings, in which:
FIG. 1 is a diagrammatic representation of an augmented reality system according to one contemplated embodiment of the current disclosure showing the augmented reality assembly, medical equipment, and information storage, retrieval, and communication system;
FIG. 2 is a diagrammatic representation of the augmented assembly of FIG. 1 showing the components of the assembly;
FIG. 3 is a diagrammatic representation of the augmented reality device and the person support structure of FIG. 1 showing an example of what a caregiver would see when using the augmented reality assembly in the patient care environment;
FIG. 4 is a side perspective view of the person support apparatus, person support surface, and control system according to one contemplated embodiment;
FIG. 5 is a partial diagrammatic representation of the person support surface of FIG. 4; and
FIG. 6 is a partial cut-away view of the person support surface of FIG. 4 showing the sensors positioned therein.

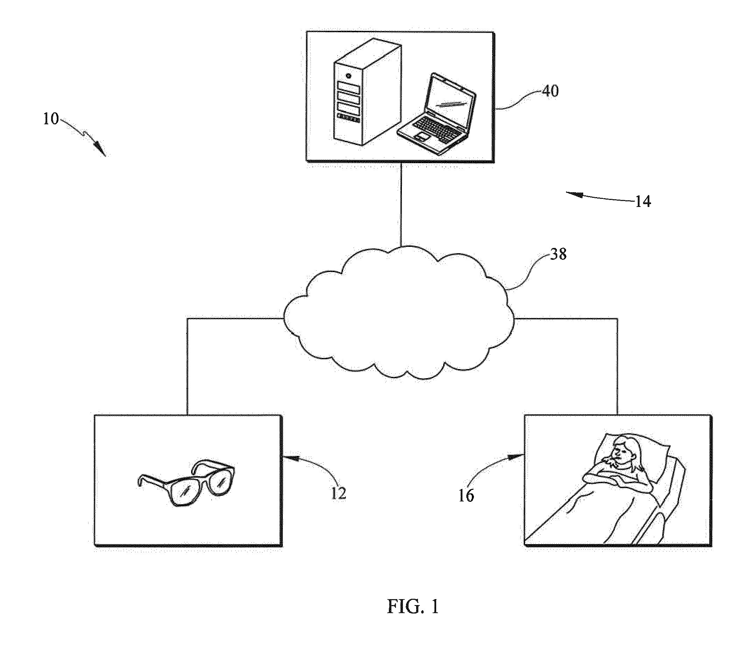

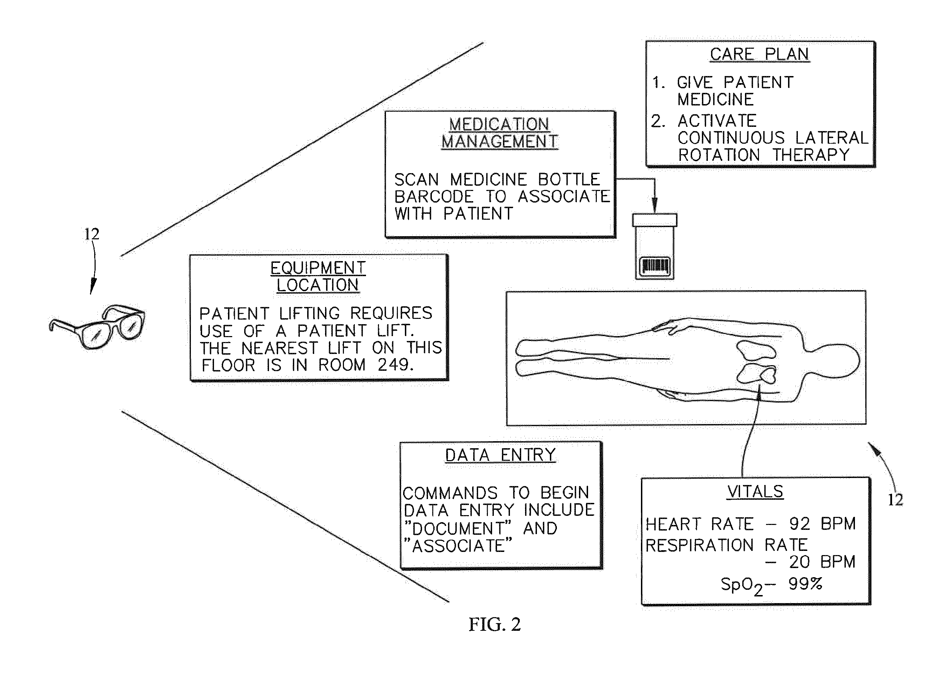

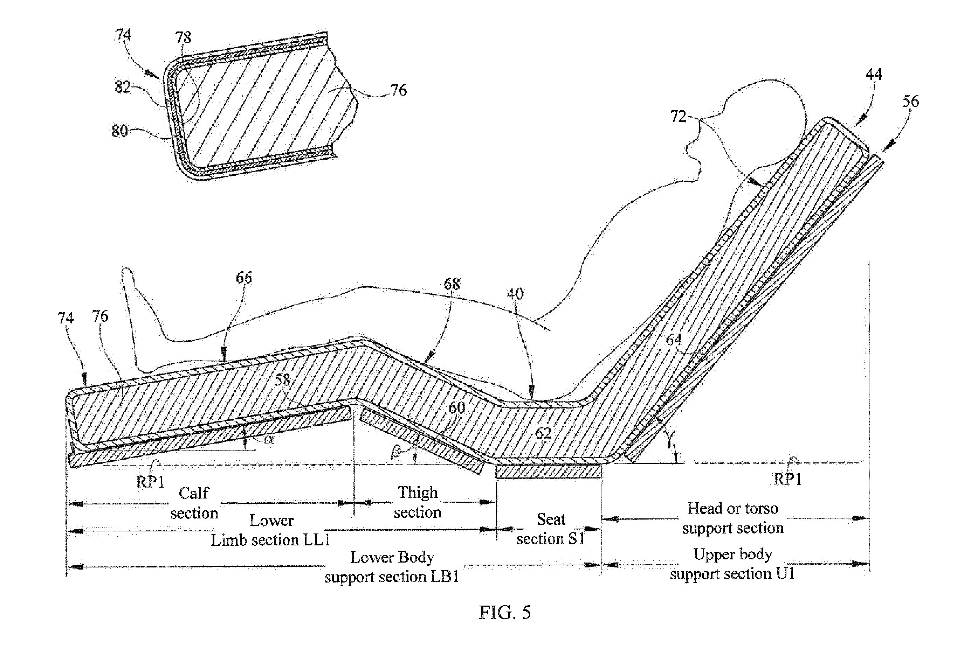

An augmented reality system 10 according to one contemplated embodiment is shown in Figs. 1-6. The system 10 is configured to assist a caregiver by displaying, among other things, information, tasks, protocols, and control options associated with the patient and/or equipment in the vicinity of the patient. The system 10 includes a user interface assembly 12, information storage, retrieval, and communication systems 14, and medical equipment 16. The system 10 is configured to, among other things, communicate information, perform tasks, and/or control other devices and systems depending on the input from the user. In one contemplated embodiment, the system 10 communicates information to the user that includes, but is not limited to, the status of the medical equipment, an object (such as, for example, a medicine container) being examined, the patient (including, but not limited to, physiological parameters, protocols, medications, actions to be taken, adverse condition predictions, and identification information), and tasks for the caregiver to perform (such as, for example, activate heat and moisture regulating therapy or scan medicine container to associate medicine with patient). In another contemplated embodiment, the user can perform tasks with the system 10, including, but not limited to, using voice activation for data entry to document pain thresholds or other observations about the patient, using voice recognition to identify patients and caregivers, and/or associating a medicine container with a patient or bed by using a barcode scanning program to scan the barcode the user is looking at. In another contemplated embodiment, the system 10 can be used by the user to control other devices or systems, such as, for example, to raise a patient using a lift system, activate a therapy on a hospital bed, dim or increase the intensity of the lights in the room, and/or call for help. In some contemplated embodiments, system 10 is also configured to provide other relevant information, including, but not limited to, information about the facility, the procedures the person will be undergoing, directions to the nearest equipment needed, location of other caregivers, and/or other information related to the patient, caregiver, medical devices, systems, and facility.

The user interface assembly 12 includes a display device 18, communication circuitry 20, a microphone 22, an audio output device 24, a camera 26, a location identification system 28, control circuitry including a processor 30 and memory 32, a power source 34, and a radio frequency reader 36. In one contemplated embodiment, the assembly 12 includes augmented reality glasses. One example of such an assembly includes the Smart Glasses M100 disclosed and marketed by Vuzix Corporation. Another example of such an assembly includes the Google Glass augmented reality glasses disclosed by Google, Inc. In some contemplated embodiments, the assembly 12 includes control buttons (not shown) integrated therein, such as, for power, volume control, display brightness or contrast, or other functions. In some contemplated embodiments, the assembly 12 also includes a projector (not shown) for projecting images onto surfaces and/or overlaying images on the patient.

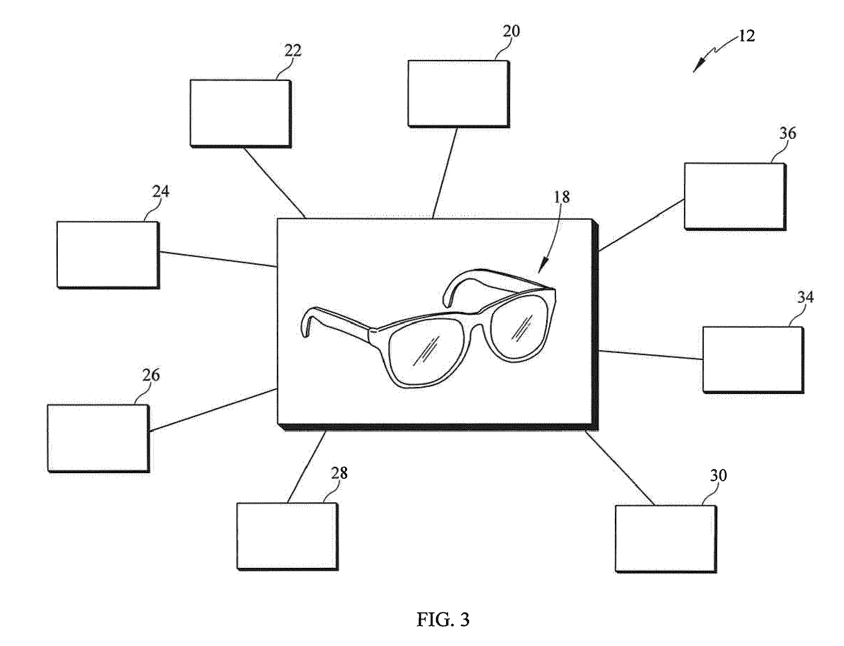

The display device 18 is configured to display information, tasks, and/or device controls. In one contemplated embodiment, the display device 18 includes an optics engine with a display resolution of WQVGA color 16X9 displays, a field of view of 16 degrees (a 4" cellphone screen at 14"), and a brightness of greater than 2000 nits. In one contemplated embodiment, the information, tasks, and/or device controls are displayed on the lens of the glasses. In another contemplated embodiment, the information, tasks, and/or device controls are projected on the user's retina, or displayed on the user's contact lens. In one contemplated embodiment, the display device 18 displays information about the status of the medical equipment 16 (i.e., bed exit alarm status, head of bed angle, battery life, active therapies, etc.), the physiological characteristics of the patient (i.e., Sp02, heart rate, respiration rate, etc.), medicine management tasks (i.e., give patient X medication, scan barcode of medicine container, etc.), care plan tasks for the caregiver (patient turn at 2:15, check blood pressure, turn on bed exit alarm, patient prep for surgery at 7:30, etc.), bed controls (raise head of bed, activate therapy, lower upper frame height, turn off bed exit alarm, etc.), or other information, tasks, or controls the caregiver might desire access to. The information, tasks, and/or device controls are displayed adjacent to the object to which it pertains. For example, as shown in FIG. 3, in the user's field of vision the physiological parameters would be displayed adjacent to a person (heart rate adjacent to the heart, identification information adjacent to the face) and medical device control options positioned adjacent to the medical device. In some contemplated embodiments, the user can customize or change what information/options/tasks are displayed and when they are displayed through voice command, using gestures, tracking a stylus or markers on fingertips, through a user's predefined profile, a hospital care protocol, or a patient care profile. In some contemplated embodiments, the information/options/tasks displayed can correspond to parameters that a hospital care protocol requires the caregiver to check for a given diagnosis, or that a predetermined diagnosis profile specifies for the patient's current diagnosis or that may be relevant to potential adverse conditions that can arise given the diagnosis, medical history, medications, level of activity, procedures performed, or other patient status or condition information.

The communication circuitry 20 is configured to communicate with the medical equipment 16 and information systems 14 using wireless communication techniques. In one contemplated embodiment, the communication circuitry 20 communicates using WiFi (i.e., WiFi 802.11 b/g/n). In another contemplated embodiment, the communication circuitry 20 communicates via Bluetooth. In other contemplated embodiments, the communication circuitry can include wired communication ports (such as, a USB or Ethernet port) that allow the assembly 12 to be directly connected to medical equipment 16 and/or computers to update the assembly 12 and/or provide additional information or control options for the medical equipment 16. In some contemplated embodiments, the communication circuitry 20 wirelessly connects (through WiFi or Bluetooth or IR or other wireless techniques) to communication circuitry on the medical equipment 16 and receives information (i.e., status information) from the medical equipment, and/or communicates information or operational commands to the medical equipment 16 to be stored or carried out by the medical equipment 16 (i.e., raise the head section of the bed to 30 degrees). In some contemplated embodiments, the communication circuitry 20 connects to the wired network in the room via a Bluetooth transmitter in the room.

The microphone 22 is configured to receive audio inputs from the user and/or record audio signals. In one contemplated embodiment, the microphone 22 is configured to receive voice commands from the user. In another contemplated embodiment, the microphone 22 is configured to record conversations between the caregiver and patient. In another contemplated embodiment, the microphone 22 is configured to be used for voice recognition. In some contemplated embodiments, the microphone 22 is used to document a patient's pain threshold after a caregiver gives the documentation command (verbally or by selecting a documenting option from the menu of options displayed on the display device 18). In some contemplated embodiments, the user can cause the medical equipment 16 to perform a function by issuing a voice command through the input, for example, activate the bed exit alarm or lower a patient lifted by a lift device so that the caregiver can use their hands to attend to the patient and hold the patient or direct the movement of the sling as it lowers.

The audio output device 24 includes a speaker that provides verbal cues to the user or can be used to communicate with a person remotely (i.e., nurse call routed to the assembly 12). In one contemplated embodiment, the audio output device enables the user to receive feedback from the assembly 12 when a command is given (i.e., when a caregiver asks the assembly to document the pain threshold, the assembly can respond by asking how much pain is being experienced on a scale of 1-10, then the assembly 12 can record the response from the user in the EMR or in the memory until it can be uploaded to the EMR). In another contemplated embodiment, the user can have a conversation with another caregiver or a patient using the assembly 12.

The camera 26 is used to identify objects in the user's field of view. In one contemplated embodiment, the camera 26 is a 720p HD camera with a 16:9 aspect ratio and video recording capability. In other contemplated embodiments, the camera 26 includes multispectral imaging capabilities (including, but not limited to, infrared and visible spectrums), which the user can use to examine a patient for wounds or for other visual assessments. In another contemplated embodiment, the camera 26 can take pictures of an object or of an identified area. In another contemplated embodiment, the camera 26 is configurable to zoom in on a desired area to display on the display 18. Zooming can be accomplished using gestures or other input techniques previously described.

The location identification system 28 is used to identify the location of the user. In one contemplated embodiment, the location identification system 28 includes a GPS system. In another contemplated embodiment, the location identification system 28 uses a program that triangulates the person's position by comparing the time it takes a signal takes to reach the user from at least two wireless access points, and/or comparing the strength of the wireless signals. In another contemplated embodiment, the system 28 is configured to track the movement of the user's head with three degree of freedom. In another contemplated embodiment, the system 28 includes an accelerometer to track movement of the assembly 12. In another contemplated embodiment, the system 28 includes a digital compass. In still another contemplated embodiment, as further described below, the location identification system 28 receives location information from an indicator, such as, a barcode, IR signal, RF signal, and/or Bluetooth signal, that is located in the care facility. In some contemplated embodiments, the location identification system 28 may not be needed where the medical equipment includes a low frequency radio transceiver configured to communicate information about the medical equipment and/or patient associated therewith when the user interface 12 is located within range of the low frequency transceiver. The location identification system 28 may also include an RFID reader configured to read RFID tags on the medical equipment, patient's wrist band, or that are located throughout the facility.

Once the system knows where the wearable user interface 12 is located, the system can direct data corresponding to the medical device and/or patient in the same location to the user interface 12 for display in the caregiver's field of vision. In some contemplated embodiments, the user interface 12 displays a list of patients that a caregiver may select from to have the data related to that patient and/or medical equipment associated with the patient directed to the user interface. Some of the information that can be sent to the user interface includes whether the medical device is clean or dirty, whether the medical device can be used with the patient, whether the caregiver is certified to use the medical device, whether the caregiver needs additional equipment, such as, a barrier gown, or needs to perform a task before using the medical device, such as, washing their hands.

The control circuitry is configured to control the operation of the assembly 12. The processor 30 is configured to execute programs stored in the memory 32 to enable the assembly 12 to perform a variety of functions. In some contemplated embodiments the programs are stored and executed on a remote device, such as, the hospital network server, and the processor 30 and memory 32 control the operation of the various components of the assembly 12 to provide the input to the remote system and to carry out functions in accordance with the output from the remote system.

The programs enable the assembly 12 to perform a number of functions that could help caregivers perform their tasks more efficiently and effectively. In one contemplated embodiment, one of the programs includes a barcode reading/scanning program that allows the user to scan a barcode on an object by positioning the barcode in front of the camera 26 or in the person's field of view. One example of such a program is RedLaser Barcode & QR Scanner sold by RedLaser, an eBay Inc. company. The assembly 12 allows the user to scan the barcode by having it in the person's field of vision, touching the barcode with a fingertip marker, pointing to it with a stylus, or using a voice command that searches for barcodes in the user's field of vision and scans them. In some contemplated embodiments, the barcode is located in a room and provides information about the user's location in a care facility. In some contemplated embodiments, the barcodes are dynamically generated on a user interface and include information about the location within the care facility and/or identification information for the patient and/or medical device proximate to the barcode. In some contemplated embodiments, the graphical user interface on the medical device generates the barcode. In another contemplated embodiment, one of the programs includes an electronic medical record (EMR) interface that allows the user to view a patient's medical information and add additional medical information (i.e., current observations, diagnoses, compliance information, or other information). One example of such a program is the drchrono EHR mobile application sold by DrChrono.com Inc. In another contemplated embodiment, one of the programs includes a facial recognition program, which can be used, among other things, to identify the patient. One example of such a program is the KLiK application developed by Face.com. Another example of a facial recognition program is Visidon AppLock by Visidon Ltd. In another contemplated embodiment, one of the programs includes a location and tracking program that could be used to locate and track caregivers or equipment. One example of such a program is the Hill-Rom® Asset Tracking solution program. Another example of a locating and tracking application is Find My Friends by Apple. In another contemplated embodiment, one of the programs includes a limb recognition and tracking program. One example of such a program is used in the Microsoft Kinect device. In another contemplated embodiment, one of the programs includes an image processing program that allows the user to digitally filter the information being received from the camera. For example, a user may wish to illuminate a patient's skin with infrared light or select wavelengths of light, and filter the reflected light to see if a pressure ulcer or deep tissue injury is forming. In another contemplated embodiment, one of the programs enables the camera 26 can locate a person's vein in their arm using infrared camera light and display it on the display device 18. In another contemplated embodiment, one of the programs enables the camera 26 can identify hot-spots where pressure ulcers might form or detect a wound that has formed or is forming using infrared thermography. In another contemplated embodiment, one of the programs includes a voice recognition program that can be used to authenticate the caregiver and/or patient. In another contemplated embodiment, one of the programs helps facilitate interaction between the caregiver and the patient by displaying data that is relevant to the question being asked so that the caregiver can review the information as they carry on the conversation. The information displayed can be dictated by the user's profile, a diagnosis profile, or the hospital care protocol, or can be filtered based on key words used by the user according to a predetermined algorithm (i.e., if you hear the word "sleep", display heart rate and respiration rate, or if you hear "trouble" and "bathroom", display the results from the recent UTI test), or can be verbally requested by the user. In another contemplated embodiment, one of the programs allows the user to take a picture of a wound, for example, in a homecare setting, and send the image to a caregiver to ask if the wound is infected. In another contemplated embodiment, one of the programs allows the user to take a picture of a wound or other condition and save it to the EMR for documentation. In another contemplated embodiment, one of the programs alerts you when you walk into the patient's room that the person is greater than 500lbs and, according to the hospital care protocol, you need to use a lift device to lift them or seek additional help before attempting to lift or reposition them. Compliance data for whether or not you used a lift to move the patient in these circumstances can also be tracked with the assembly 12. In another contemplated embodiment, one of the programs locates the nearest lift device capable of lifting the patient (on your current floor and/or anywhere in the care facility) when the hospital protocol dictates that the person should be lifted by a lift, and gives you directions to the lift. In another contemplated embodiment, one of the programs is configured to visually identify the medication being given to the patient (by the physical features of the pill or from the barcode on the medicine bottle) and alert the caregiver if the medication is the wrong medication or if the patient is not due to receive the medication yet. In another contemplated embodiment, one of the programs can use facial recognition to alert the caregiver if the person on the hospital bed is not the person that is assigned to the bed. In some contemplated embodiments, one of the programs can display a red X (and/or present an audible message) before the caregiver enters the room to indicate that the patient is in quarantine and the caregiver needs to take precautions. In another contemplated embodiment, one of the programs can utilize limb recognition so that a processed image (i.e., an infrared image, thermal image, or x-ray image) can be overlaid on the patient's body. One example of a program projecting images onto the patient is VeinViewer® developed by Christie Digital Systems USA, Inc. In some contemplated embodiments, one of the programs causes information, such as, a task list or nurse call request, for a specific patient to be displayed upon reaching the patient's room. In another contemplated embodiment, one of the programs causes information to be displayed once you are within a predetermined distance of the patient. In another contemplated embodiment, one of the programs recognizes other medical equipment (i.e., an SCD pump or a patient lift) in the room based on its appearance (i.e., using computer vision techniques) by comparing the appearance of the device to a library of medical device images. In another contemplated embodiment, one of the programs can identify the patients based on the hospital beds in the room and the user can select which patient's information they want to view. In another contemplated embodiment, one of the programs enables a user to receive a nurse call and activate a video camera in the room where the nurse call signal originated so that the caregiver can view the status of the room en route to the room. In another contemplated embodiment, one of the programs analyzes the patient's physiological information and predicts when an adverse event might occur. One example of such a program is the Visensia program offered by OBS Medical Ltd. In another contemplated embodiment, one of the programs displays the adverse event analysis on the display device 18 and can activate/provide alerts to the caregiver via the display device or an audible alert when an adverse event is predicted to occur within a predetermined amount of time. In another contemplated embodiment, one of the programs can allow the user to scroll through a list of names for the patient, medications or medical devices seen in the room and pick the corresponding image to confirm the identity. In another contemplated embodiment, one of the programs utilizes an overhead camera in the patient's room to record their sleep history and play a time-lapse video back for the caregiver to see the patient's activity while sleeping (or whether or not the patient needs to be repositioned because they have been inactive while they are awake). In another contemplated embodiment, one of the programs displays a patient's EEG readings in a menu adjacent to their heart and the user can select the menu to read the EEG chart.

The power source 34 is integrated into the assembly 12 and provides power to the various components. In one contemplated embodiment the power source is a battery that is capable of providing up to about 8 hours of power to the assembly 12. In some contemplated embodiments, the power source 34 is charged using a wired connection (i.e., though contacts or a plug) or a wireless connection (i.e., inductive charging).

The radio frequency reader 36 is integrated into the assembly 12 and is configured to read radio frequency tags (not shown). In one contemplated embodiment, the reader 36 is used to read a patient's RFID bracelet. In some contemplated embodiments, the barcode scanner is used to scan the barcode on the patient's ID bracelet. In another contemplated embodiment, the reader 36 is used to read the RFID tag on the medicine container. In another contemplated embodiment, the reader 36 is used to read the RFID tag on other medical equipment. In other contemplated embodiments, the reader 36 is used to read RFID tags to associate objects with one another (i.e., a medication and a patient and/or medical equipment and a patient).

The information system 14 includes a hospital network 38 with servers 40, such as, an electronic medical records database or server. The communication system 14 is configured to provide the assembly 12 with information about the patient's medical history, the location of the user, care protocols, patient care tasks, and other information about the caregiver, patient, facility, and medical equipment. In some contemplated embodiments, the system 14 includes patient stations capable of generating hospital calls and a remote master station which prioritizes and store the calls. One example of such a system is disclosed in U.S. Pat. No. 5,561,412 issued on Oct. 1, 1996 to Novak et al. Another example of such a system is disclosed in U.S. Pat. No. 4,967,195 issued on May 8, 2006 to Shipley.

In some contemplated embodiments, the system 14 includes a system for transmitting voice and data in packets over a network with any suitable number of intra-room networks that can couple a number of data devices to an audio station, where the audio station couples the respective intra-room network to a packet based network. One example of such a system is disclosed in U.S. Pat. No. 7,315,535 issued on Jan. 1, 2008 to Schuman. Another example of such a system is disclosed in U.S. Patent Publication No. 2008/0095156 issued on Apr. 24, 2008 to Schuman.

In other contemplated embodiments, the system 14 includes a patient/nurse call system, a nurse call/locating badge, an EMR database, and one or more computers programmed with work-flow process software. One example of such a system is disclosed in U.S. Patent Publication No. 2008/0094207 published on Apr. 24, 2008 to Collins, Jr. et al. Another example of such a system is disclosed in U.S. Patent Publication No. 2007/0210917 published on Sep. 13, 2007 to Collins, Jr. et al. Yet another example of such a system is disclosed in U.S. Pat. No. 7,319,386 published on Jan. 15, 2008 to Collins, Jr. et al. It should be appreciated that the workflow process software can be the NaviCare® software available from Hill-Rom Company, Inc. It should also be appreciated that the workflow process software can be the system disclosed in U.S. Pat. No. 7,443,303 issued on Oct. 28, 2008 to Spear et al. It should further be appreciated that the badge can be of the type available as part of the ComLinx® system from Hill-Rom Company, Inc. It should also be appreciated that the badge can also be of the type available from Vocera Communications, Inc.

In other contemplated embodiments, the system 14 is configured to organize, store, maintain and facilitate retrieval of bed status information, along with the various non-bed calls placed in a hospital wing or ward, and remotely identify and monitor the status and location of the person support apparatus, patients, and caregivers. One example of such a system is disclosed in U.S. Pat. No. 7,242,308 issued on Jul. 10, 2007 to Ulrich et al. It should be appreciated that the remote status and location monitoring can be the system disclosed in U.S. Pat. No. 7,242,306 issued on Jul. 10, 2007 to Wildman et al. It should also be appreciated that the remote status and location monitoring can be the system disclosed in U.S. Patent Publication No. 2007/0247316 published on Oct. 25, 2007 to Wildman et al.

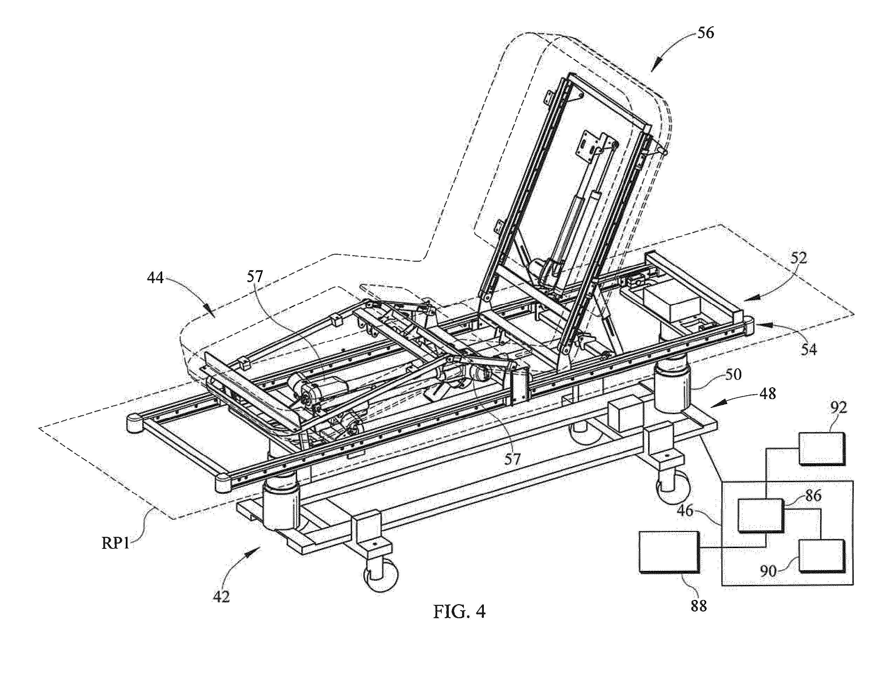

Medical equipment 16 includes a number of medical devices and systems used with patients. Some of the medical devices include, airway clearance systems (chest wall oscillation, sequential compression, cough assist, or other devices), person support structures or hospital beds, person lift systems (mobile lift systems, wall mounted lift systems, and/or ceiling lift systems), respirators, infusion pumps, IV pumps, or other medical devices. The person support structure includes a person support frame 42, a person support surfaces 44, and the associated control systems 46. The surface 44 (or mattress 44) is supportable on the frame 42 as shown in FIG. 4-5, and the control systems 46 are configured to control various functions of one or both of the frame 42 and the surface 44. In some contemplated embodiments, the person support structure can be a stretcher, an operating room table, or other person supporting structure.

The frame 42 includes a lower frame 48, supports 50 or lift mechanisms 50 coupled to the lower frame 48, and an upper frame 52 movably supported above the lower frame 48 by the supports 50. The lift mechanisms 50 are configured to raise and lower the upper frame 52 with respect to the lower frame 48 and move the upper frame 52 between various orientations, such as, Trendellenburg and reverse Trendellenburg.

The upper frame 52 includes an upper frame base 54, a deck 56 coupled to the upper frame base 54, a plurality of actuators 57 coupled to the upper frame base 54 and the deck 56, a plurality of siderails (not shown), and a plurality of endboards (not shown). The plurality of actuators 57 are configured to move at least a portion of the deck 56 along at least one of a longitudinal axis, which extends along the length of the upper frame 52, and a lateral axis, which extends across the width of the upper frame 52, between various articulated configurations with respect to the upper frame base 54. The deck 56 includes a calf section 58, a thigh section 60, a seat section 62, and a head and torso section 64. The calf section 58 and the thigh section 60 define a lower limb support section LL1. The head and torso section 64 define an upper body support section U1. The seat section 62 defines the seat section S1. The calf section 58, the thigh section 60, and the seat section 62 define a lower body support section LB1. At least the calf section 58, the thigh section 60, and the head and torso section 64 are movable with respect to one another and/or the upper frame base 54. In some contemplated embodiments, the calf section 58, the thigh section 60, the seat section 62, and the head and torso section 64 cooperate to move the frame 42 between a substantially planar or lying down configuration and a chair configuration. In some contemplated embodiments, the calf section 58, the thigh section 60, the seat section 62, and the head and torso section 64 cooperate to move the frame 42 between a substantially planar or lying down configuration and an angled or reclined configuration. In some contemplated embodiments, the head and torso section 64 is moved such that it is at an angle of at least about 30° with respect to a reference plane RP1 passing through the upper frame 52.

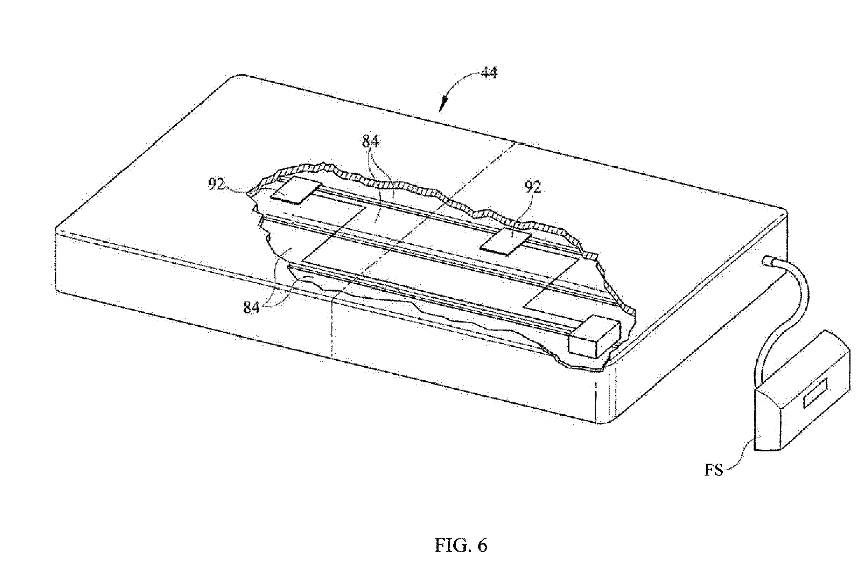

The surface 44 is configured to support a person thereon and move with the deck 56 between the various configurations. In some contemplated embodiments, the surface 44 is a hospital bed mattress 44. In some contemplated embodiments, the surface 44 is a consumer mattress. The surface 44 includes a calf portion 66, a thigh portion 68, a seat portion 70, and a head and torso portion 72, which is supported on corresponding sections of the deck 56. In one illustrative embodiment, the deck sections help move and/or maintain the various portions of the mattress 44 at angles α, β and y with respect to the reference plane RP1. In some contemplated embodiments, the surface 44 is a non-powered (static) surface. In some contemplated embodiments, the surface 44 is a powered (dynamic) surface configured to receive fluid from a fluid supply FS1 as shown in Fig. 6.

The surface 44 includes a mattress cover 74 and a mattress core 76. In some contemplated embodiments, the surface 44 includes a temperature and moisture regulating topper (not shown) coupled to the mattress cover 74. The mattress cover 74 encloses the mattress core 76 and includes a fire barrier 78, a bottom ticking 80 or durable layer 80, and a top ticking 82. In some contemplated embodiments, the fire barrier 78 is the innermost layer of the cover 74, the top ticking 82 is the outermost layer, and the bottom ticking 80 is positioned between the fire barrier 78 and the top ticking 82 and is not coupled to the top ticking 82. The bottom ticking 80 and the top ticking 82 are vapor and air impermeable. In some contemplated embodiments, the top ticking 82 and the bottom ticking 80 are composed of polyurethane coated nylon and the bottom ticking 80 is configured to facilitate movement of the top ticking 82 with respect to the fire barrier 78. In other contemplated embodiments, the top ticking 82 and/or the bottom ticking 80 can be air and/or moisture permeable.

The mattress core 76 can be composed of a single type of material or a combination of materials and/or devices. In the case of a powered surface, the mattress core 76 includes at least one fluid bladder 84 therein that receives fluid from a fluid supply (not shown) to maintain the fluid pressure within the fluid bladder 84 at a predetermined level. In some contemplated embodiments, the powered surface can include non-powered components, such as, a foam frame that at least one fluid bladder 84 is positioned between. In some contemplated embodiments, a fluid bladder 84 can be positioned proximate to the thigh section and inflated or the calf portion 66, thigh portion 68, and/or seat portion 70 (including their corresponding deck sections) can be articulated to help prevent the occupant from sliding down the mattress 44 as, for example, the inclination of the head and torso section 64 increases with respect to the reference plane RP1. In some contemplated embodiments, wedge shaped bladders are mirrored laterally about the centerline of the mattress 44 and are configured to be inflated consecutively to laterally tilt the occupant, thereby relieving pressure on various portions of the occupant's body to help reduce the occurrences of pressure ulcers.

In the case of a non-powered surface, the mattress core 76 is composed of a cellular engineered material, such as, single density foam. In some contemplated embodiments, the mattress core 76 includes at least one bladder 84, such as, a static air bladder or a static air bladder with foam contained there within, a metal spring and/or other non-powered support elements or combinations thereof. In some contemplated embodiments, the mattress core 76 and includes multiple zones with different support characteristics configured to enhance pressure redistribution as a function of the proportional differences of a person's body. Also, in some embodiments, the mattress core 76 includes various layers and/or sections of foam having different impression load deflection (ILD) characteristics, such as, in the NP100 Prevention Surface, AccuMax Quantum™ VPC Therapy Surface, and NP200 Wound Surfaces sold by Hill-Rom®.

The control system 46 is configured to change at least one characteristic of the frame 42 and/or surface 44 in accordance with a user input. In one contemplated embodiment, the control system 46 controls the operation of the fluid supply FS1 and the actuators 57 to change a characteristic of the surface 44 and frame 42, respectively. The control system 46 includes a processor 86, an input 88, memory 90, and communication circuitry 91 configured to communicate with the communication circuitry 20 and/or the hospital network 38. In some contemplated embodiments, the input 88 is a sensor 92, such as, a position sensor, a pressure sensor, a temperature sensor, an acoustic sensor, and/or a moisture sensor, configured to provide an input signal to the processor 86 indicative of a physiological characteristic of the occupant, such as, the occupant's heart rate, respiration rate, respiration amplitude, skin temperature, weight, and position. In some contemplated embodiments, the sensors 92 are integrated into the mattress cover 74, coupled to the frame 42 (i.e., load cells coupled between the intermediate frame and the weigh frame, which form the upper frame base 54), coupled to other medical devices associated with or in communication with the control system 46, and/or are coupled to the walls or ceiling of the room or otherwise positioned above the bed (i.e., an overhead camera for monitoring the patient). In some contemplated embodiments, the sensor 92 can be contactless (i.e., positioned in the mattress) or can be attached to the patient (i.e., SpO2 finger clip or EEG electrode attached to the person's chest). In some contemplated embodiments, the input 88 is a user interface configured to receive information from a caregiver or other user. In other contemplated embodiments, the input 88 is the EMR system in communication with the processor 86 via the hospital network 14. In some contemplated embodiments, the processor 86 can output information, automatically or manually upon caregiver input, to the EMR for charting, which can include therapy initiation and termination, adverse event occurrence information, therapy protocol used, caregiver ID, and any other information associated with the occupant, caregiver, frame 42, surface 44, and adverse event.

The memory 90 stores one or more instruction sets configured to be executed by the processor 86. The instruction sets define procedures that cause the processor 88 to implement one or more protocols that modify the configuration of the frame 42 and/or mattress 44.

Many other embodiments of the present disclosure are also envisioned. For example, an augmented reality system comprises a user interface system, a care facility network, and a medical device. The network is in communication with the user interface system. The medical device is configured to be used with a patient and is in communication with the user interface system. The user interface system receives information from the care facility network and the medical device and displays the information in a user's field of vision.

Any theory, mechanism of operation, proof, or finding stated herein is meant to further enhance understanding of principles of the present disclosure and is not intended to make the present disclosure in any way dependent upon such theory, mechanism of operation, illustrative embodiment, proof, or finding. It should be understood that while the use of the word preferable, preferably or preferred in the description above indicates that the feature so described may be more desirable, it nonetheless may not be necessary and embodiments lacking the same may be contemplated.

While embodiments of the disclosure have been illustrated and described in detail in the drawings and foregoing description, the same are to be considered as illustrative and not intended to be exhaustive or to limit the disclosure to the precise forms disclosed. Additional alternatives, modifications and variations may be apparent to those skilled in the art. Also, while multiple inventive aspects and principles may have been presented, they need not be utilized in combination, and various combinations of inventive aspects and principles are possible in light of the various embodiments provided above.

Embodiments of the invention can be described with reference to the following numbered clauses, with preferred features laid out in the dependent clauses:
1. An augmented reality system, comprising:
   a medical device including a control system; and
   a user interface assembly configured to display information related to the control system of the medical device in the user's field of vision.
2. The system of clause 1, wherein the user interface assembly includes augmented reality glasses.
3. The system of clause 1, wherein the user interface assembly includes a display positionable in a person's field of vision.
4. The system of clause 3, wherein the display includes a contact lens.
5. The system of clause 1, wherein the user interface assembly includes a projector configured to project the image on the user's retina.
6. The system of clause 1, wherein the user interface assembly includes a display configure
7. The system of clause 1, wherein the medical device is a hospital bed configured to support an occupant thereon and the control system includes sensors configured to sense at least one physiological parameter of the occupant, the user interface assembly being configured to display at least one of the physiological parameters of the occupant.
8. The system of clause 1, wherein the medical device is a hospital bed configured to support an occupant thereon, the user interface assembly being configured to display the status of the hospital bed.
9. The system of clause 1 further comprising a hospital network system, the user interface assembly being configured to display information provided by the hospital network system to the user interface assembly in the user's field of view.
10. The system of clause 9, wherein the information includes a patient's medical records.
11. The system of clause 9, wherein the information includes a care facility's care protocol.
12. The system of clause 9, wherein the information includes a patient's care plan.
13. The system of clause 9, wherein the information includes a task list.
14. The system of clause 9, wherein the user input assembly includes an input device configured to receive information from the user and communicate the information to a storage location in communication with the hospital network.
15. The system of clause 9, wherein information about a patient is displayed adjacent to the patient when the patient is in the caregiver's field of vision.
16. The system of clause 1, wherein the wherein the information is displayed adjacent to the source of the information.
17. An augmented reality system, comprising:
   a care facility network; and
   a user interface assembly configured to display information communicated to the user interface assembly by the care facility network in the user's field of vision.
18. The system of clause 17, wherein the information includes a patient's medical records.
19. The system of clause 17, wherein the information includes a care facility's care protocol.
20. The system of clause 17, wherein the information includes a patient's care plan.
21. The system of clause 17, wherein the information includes a task list.
22. The system of clause 17, wherein the user input assembly includes an input device configured to receive information from the user and communicate the information to a storage location in communication with the hospital network.
23. The system of clause 17, wherein information about a patient is displayed adjacent to the patient when the patient is in the caregiver's field of vision.
24. An augmented reality system, comprising:
   a display device;
   communication circuitry configured to send and receive information from an information source;
   a controller configured to control the display device to display information received from the information source in a user's field of vision.
25. The system of clause 24 further comprising an image capture device configured to capture at least one image representative of the user's field of vision.
26. The system of clause 25, wherein the image capture device is a video camera configured to record what is in the user's field of vision.
27. The system of clause 24 further comprising a radio frequency reader configured to read radio frequency tags.
28. The system of clause 24 further comprising an audio output device.
29. The system of clause 24 further comprising an audio input device.
30. The system of clause 24 further comprising a GPS location system.
31. An augmented reality system, comprising:
   a user interface system;
   a care facility network in communication with the user interface system; and
   a medical device configured to be used with a patient and is in communication with the user interface system, wherein the user interface system receives information from the care facility network and the medical device and displays the information in a user's field of vision.
32. The system of clause 31, wherein the information corresponds to the patient's physiological characteristics.
33. The system of clause 31, wherein the information corresponds to the patient's medical history.
34. The system of clause 31, wherein the information corresponds to a status of the medical device.
35. The system of clause 31, wherein the information corresponds to a care facility protocol.
36. The system of clause 31, wherein the user interface system includes at least one of a display, a camera, a barcode scanner, a GPS system, an audio input, an audio output, and a controller.
37. The system of clause 36, wherein the controller and the camera cooperate to identify a person in the user's field of vision.
38. The system of clause 36, wherein the controller and the GPS system cooperate to identify the location of the user.
39. The system of clause 36, wherein the controller and one of the barcode scanner and the RFID scanner cooperate to associate medical equipment and objects with the patient.
40. The system of clause 36, wherein the controller is configured to interface with an EMR system.
41. The system of clause 36, wherein the camera is configured to record images in the visual and infrared light spectrums.
42. The system of clause 36, wherein the controller is configured to apply image processing techniques to images received from the camera.
43. The system of clause 36, wherein the audio input is configured to receive voice commands that cause the controller to perform a function in accordance therewith.
44. An information communication system, comprising:
   a wearable user interface configured to display information it receives in the user's field of vision; and
   a medical device including communication circuitry configured to communicate information related to the medical device and/or a patient associated with the medical device to the wearable user interface when the wearable user interface enters a communication zone proximate to the medical device.
45. The system of clause 44, wherein the medical device communicates information to the wearable user interface using a low frequency radio signal.
46. The system of clause 44, wherein the information related to the medical device includes one or more of an operational status of the medical device, a cleaning status of the medical device, a use protocol for the medical device, a status of a therapy, and a control option for controlling the medical device.
47. The system of clause 44, wherein the information related to the patient includes one or more of a characteristic of the patient, a medical history of the patient, a care plan for the patient, and a task list.
48. The system of clause 44, wherein the wearable user interface includes glasses and the information is displayed on at least one lens of the glasses.
49. The system of clause 44, wherein the wearable user interface is configured to project the information onto the user's retina.
50. The system of clause 44, wherein the information is positioned adjacent to the medical device or patient to which it corresponds in the person's field of vision.
51. An information communication system, comprising:
   a wearable user interface configured to display information it receives in the user's field of vision;
   a medical device; and
   a communication cable configured to communicatively couple the wearable user interface with the medical device so that the wearable user interface can receive information related to one or more of the medical device and a patient associated with the medical device to be displayed in the user's field of vision.
52. An information communication system, comprising:
   a wearable user interface configured to display information it receives in the user's field of vision;
   an indicator including information related to one or more of a medical device or a patient located proximate to the indicator;
   an input device configured to receive information from the indicator;
   communication circuitry configured to be communicatively coupled with one or more of a database and a medical device to receive information related to one or more of a patient and the medical device; and
   a control system configured to cause the communication circuitry to communicatively couple with one or more of the database and the medical device in response to the information from the indicator and for the wearable user interface to display the information related to one or more of the patient and the medical device in the user's field of vision.
53. An information system configured to provide information to a user, comprising:
   a medical information database;
   a medical device associated with a patient;
   communication circuitry configure to be communicatively coupled to one or more of the medical information database and the medical device; and
   a wearable user interface configured to display information and control options in a user's field of vision, wherein the control option includes a list of patients the user may select from; and
   a control system causing the communication circuitry to communicatively couple with one or more of the medical information database and the medical device in response to the user's selection, there control system causing the wearable user interface to display information related to one or more of the selected patient and the medical device associated with the selected patient in the user's field of vision.
54. The system of any preceding clause, wherein the information related to the person includes a physiological characteristic of the person, a medical history of the person, a care plan for the person, an adverse condition assessment for the person, at least one task for a caregiver to perform, identification information for the person, medicine management tasks, protocols for the person,
55. The system of any preceding clause, wherein the information related to the medical device includes an operational status of the medical device, a control option for the medical device, a cleaning status of the medical device, a use protocol for the medical device, care plan tasks, and a status of a therapy.
56. The system of any preceding clause, wherein the control options include one or more of controlling the operation of the medical device, data entry, voice recognition, barcode scanning, device and/or patient association.
57. The system of any preceding clause, wherein the location system includes GPS.
58. The system of any preceding clause, wherein the control system includes a processor and memory configured to store a set of instructions to be selectively executed by the processor to cause the wearable user interface to perform a task.
59. The system of any preceding clause, wherein the program includes one or more of facial recognition, item or person locating and tracking, barcode reading, limb recognition, image processing, hot spot locating, vein locating, voice recognition, compliance monitoring, equipment locating, medication identification, and device and person association.
60. The system of any preceding clause, wherein the location system includes an input device configured to read an indicator located in the vicinity of the person or medical device, the indicator including information related to the location of the indicator within a care facility.
61. The system of any preceding clause, wherein the location system includes an input device configured to read an indicator on the person or medical device, the indicator including information related to the location of the indicator within a care facility.
62. The system of any preceding clause, wherein the medical device is a person support structure configured to support a person thereon.
63. The system of any preceding clause, wherein the person support structure includes sensors configured to sense at least one characteristic of a user positioned thereon.
64. The system of any preceding clause, wherein the wearable interface includes glasses and the information is displayed on a lens of the glasses.

## Claims

1. An information communication system, comprising:
a location database including location information corresponding to the location of one or more of a person and a medical device in a care facility;
communication circuitry configured to be communicatively coupled with one or more of the location database, the medical device, and an electronic medical record database;
a wearable user interface configured to display information from one or more of the location database, the medical device, and the electronic medical record database in the user's field of vision;
a location system configured to determine the location of the wearable user interface in a care facility; and
a control system configured to determine which person and/or medical device is proximate to the wearable user interface as a function of the location information from the location database and the location of the wearable user interface, the control system causing the communication circuitry to communicatively couple with one or more of the electronic medical record database and the medical device to receive information related to one or more of the person and the medical device.

2. The system of claim 1, wherein the information related to the person includes a physiological characteristic of the person, a medical history of the person, a care plan for the person, an adverse condition assessment for the person, at least one task for a caregiver to perform, identification information for the person, medicine management tasks, protocols for the person,

3. The system of either claim 1 or claim 2, wherein the information related to the medical device includes an operational status of the medical device, a control option for the medical device, a cleaning status of the medical device, a use protocol for the medical device, care plan tasks, and a status of a therapy.

4. The system of claim 3, wherein the control options include one or more of controlling the operation of the medical device, data entry, voice recognition, barcode scanning, device and/or patient association.

5. The system of any preceding claim, wherein the location system includes GPS.

6. The system of any preceding claim, wherein the control system includes a processor and memory configured to store a set of instructions to be selectively executed by the processor to cause the wearable user interface to perform a task.

7. The system of claim 6, wherein the program includes one or more of facial recognition, item or person locating and tracking, barcode reading, limb recognition, image processing, hot spot locating, vein locating, voice recognition, compliance monitoring, equipment locating, medication identification, and device and person association.

8. The system of any preceding claim, wherein the location system includes an input device configured to read an indicator located in the vicinity of the person or medical device, the indicator including information related to the location of the indicator within a care facility.

9. The system of any preceding claim, wherein the location system includes an input device configured to read an indicator on the person or medical device, the indicator including information related to the location of the indicator within a care facility.

10. The system of any preceding claim, wherein the control system includes a controller and a camera which cooperate to identify a person in the user's field of vision.

11. The system of any preceding claim, wherein the control system includes a controller and a GPS system which cooperate to identify the location of the user.

12. The system of any preceding claim, wherein the control system includes a controller and one of a barcode scanner and a RFID scanner which cooperate to associate medical equipment and objects with the person.

13. The system of any preceding claim, wherein the medical device is a person support structure configured to support a person thereon.

14. The system of claim 13, wherein the person support structure includes sensors configured to sense at least one characteristic of a user positioned thereon.

15. The system of any preceding claim, wherein the wearable interface includes glasses and the information is displayed on a lens of the glasses.
